(19) Europäisches Patentamt
European Patent Office
Office européen des brevets

(11) **EP 3 836 905 B1**

## (12) EUROPEAN PATENT SPECIFICATION

(45) Date of publication and mention
of the grant of the patent:
**04.10.2023  Bulletin 2023/40**

(21) Application number: **19752191.7**

(22) Date of filing: **13.08.2019**

(51) International Patent Classification (IPC):
**A61K 9/28** (2006.01)      **A61K 9/48** (2006.01)

(52) Cooperative Patent Classification (CPC):
**A61K 9/2866; A61K 9/2886; A61K 9/4891**

(86) International application number:
**PCT/EP2019/071667**

(87) International publication number:
**WO 2020/035475 (20.02.2020 Gazette 2020/08)**

(54) **NOVEL ORAL COMPOSITION**

NEUARTIGE ORALE ZUSAMMENSETZUNG

NOUVELLE COMPOSITION ORALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **14.08.2018  EP 18188848**

(43) Date of publication of application:
**23.06.2021  Bulletin 2021/25**

(73) Proprietor: **Apillet APS**
**4000 Roskilde (DK)**

(72) Inventors:
• **PETERSEN, Carsten Lunde**
**4000 Roskilde (DK)**
• **CHRISTENSEN, Anders**
**4000 Roskilde (DK)**

(74) Representative: **Nex & Phister Law & IP Aps
Ole Maaløes Vej 3
2200 Copenhagen N (DK)**

(56) References cited:
**WO-A1-2008/150426      WO-A1-2017/168426
US-A1- 2016 199 303**

• **KROEGEL I ET AL: "EVALUATION OF AN
ENZYME-CONTAINING CAPSULAR SHAPED
PULSATILE DRUG DELIVERY SYSTEM",
PHARMACEUTICAL RESEARCH, SPRINGER
NEW YORK LLC, US, vol. 16, no. 9, 1 January 1999
(1999-01-01), pages 1424-1429, XP000949960,
ISSN: 0724-8741, DOI: 10.1023/A:1018959327311**

**Description**

**Technical field**

The present invention relates to a solid oral composition for targeted release in an intestine of a mammal. Furthermore, the present invention relates to the use of a pair of components for preparing a coating for a solid oral dosage form.

**Background Art**

Oral delivery of active pharmaceutical ingredients is often the chosen route of delivery since it is easier, more convenient and generally painless, resulting in larger patient adherence relative to other means of delivery.

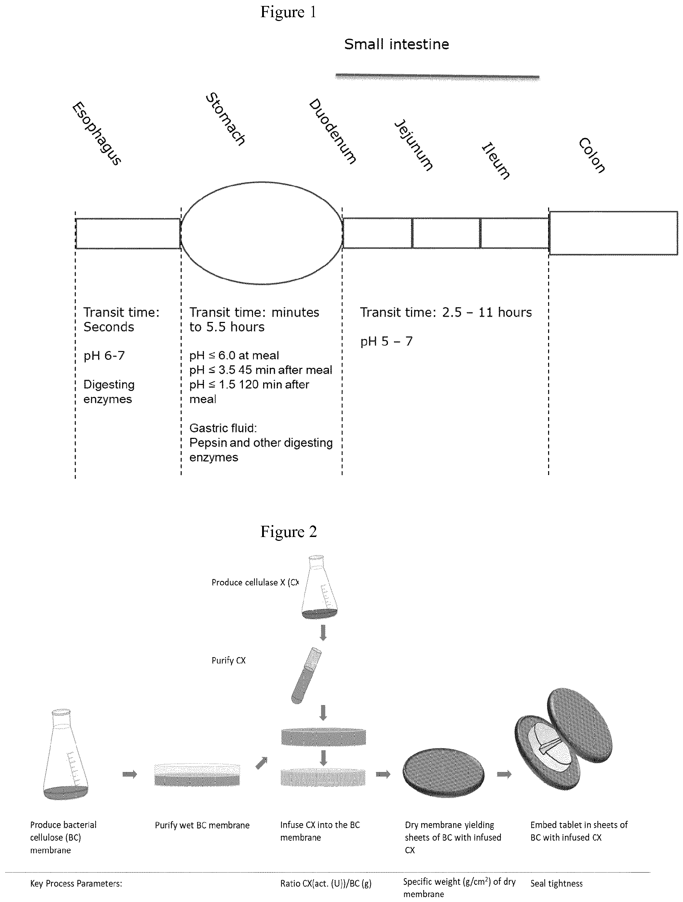

Therapeutically active and other important biological compounds may be administered to patients in a variety of ways, including for example, by the oral route. To administer proteins and peptides, but also sensitive small molecule therapeutics by the oral route is a challenge and has been pursued for many years. Some of the challenges include, but are not limited to, digestive enzymes and the strongly acidic environment of the stomach, components of pancreatic juices and secretions of the biliary system. Because of these challenges an unreliable bioavailability may be the consequence if sensitive active pharmaceutical ingredients are orally delivered. In addition, the compositions and concentrations of the gastro intestinal (GI) components are not uniform but vary within the segments of the GI tract. Some of the challenges for peptides, proteins and other sensitive substances in the GI tract are depicted in figure 1.

One skilled in the art will appreciate that, to increase efficiency and bioavailability, the dosage form should deliver the active agent (entrapped material), and any required excipient, to the part of the GI tract best suited chemically and physically for absorption of that active ingredient. There is a need for enteric coatings that can provide suitable bioavailability of such administered proteins, peptides and small molecules.

Kroegel I et al: "EVALUATION OF AN ENZYME-CONTAINING CAPSULAR SHAPED PULSATI LE DRUG DELIVERY SYSTEM", PHARMACEUTICAL RESEARCH, SPRINGER NEW YORK LLC, US, vol. 16, no. 9, 1 January 1999 (1999-01-01), pages 1424-1429, discloses an enzymatically controlled pulsatile drug release system based on an impermeable capsule body, which contains the drug and is closed by an erodible pectin/pectinase-plug. The plug was prepared by direct compression of pectin and pectinase in different ratios. In addition to the disintegration times of the plugs, the lag times and the release profiles of the pulsatile system were determined as a function of pectin: enzyme ratio, the pH of the surrounding medium, and the addition of buffering or chelating agents. The disintegration lime of the plug, respectively the lag time prior to the drug release was controlled by the pectin/enzyme ratio and the plug weight. The inclusion of a buffering agent within the plug lead to a plug disintegration independent of the surrounding pH. A pulsatile drug delivery system based on an erodible pectin plug containing a pectinolytic enzyme was developed. The drug release was controlled by the enzymatic degradation and dissolution of pectin.

US 2016/199303 A1 discloses modified release solid oral composition comprising: (a) a core comprising: (i) an effective amount of active pharmaceutical ingredient, (ii) a pH lowering agent, (iii) an absorption enhancer, (iv) a filler comprising an hydrogel-forming polymer, and (v) less than 10% by weight of disintegrant; and (b) an enteric coating surrounding the core, wherein the composition provides a pharmacokinetic profile for the active agent with a Tlag greater than 1.0 h and less than 16 h post-administration and a Tmax greater than (Tlag + 0.5 h) and less than 20 h post-administration.

WO2017/168426 A1 discloses a solid pharmaceutical formulation surrounded by a first coating and a second coating wherein said coating layer include materials which are insoluble in acidic, or enzymatic conditions (such as in the stomach) but are rapidly dissolved/ disintegrated under the enzymatic and/or mechanical grinding conditions of the small intestines or the colon.

WO2008/150426 A1 discloses a pharmaceutical composition for oral delivery of a physiologically active peptide agent comprising: (A) said peptide agent; (B) at least one pharmaceutically acceptable acid wherein acid is present in said pharmaceutical composition in a quantity which, if said composition were added to 10 milliliters of 0.1 M aqueous sodium bicarbonate solution, would be sufficient to lower the pH of said solution to no higher than 5.5; (C) an acid resistant protective vehicle effective to transport said pharmaceutical composition through the stomach of a patient while preventing contact between said active peptide agent and stomach proteases; and (D) a water soluble barrier layer that separates said acid from said protective vehicle; wherein either (a) said barrier layer adds 3-6% to the weight of the pharmaceutical composition, exclusive of any acid-protective vehicle, or (b) said barrier layer comprises a material having water solubility in excess of 11 grams per 100 milliliters of water at room temperature, or (c) said peptide agent and said acid are in the same or only layer of said composition.

## Summary of the invention

In a first aspect the present invention relates to a solid oral composition for targeted release in an intestine of a mammal comprising a core and a coating completely surrounding the core, wherein the core comprises an entrapped material to be released in the intestine, and wherein the coating or part of the coating comprises a first and a second component, wherein the first component is resistant to the environment in the stomach of a mammal and the second component enzymatically digest the first component when subjected to the more basic environment of the intestine compared to the more acidic environment of the stomach.

In a further embodiment the coating is adapted to resist breakdown from the environment in the stomach of a mammal.

In a still further embodiment the coating prevents the release of the entrapped material in the stomach of a mammal.

In a further embodiment the coating protects the entrapped material in the stomach of a mammal.

In a still further embodiment the coating is adapted to release the entrapped material in an intestine of a mammal.

As mentioned above the coating comprises a pair of components, in particular the pair of components are in contact with each other in the coating. When the coating consists of two components such components are in contact with each other, with no intermediate layer, to be ready to initiate the reaction in the intestine as explained in detail herein.

In a further embodiment the digesting activity of the second component is inhibited in the environment in the stomach of a mammal.

In a further embodiment the entrapped material is a protein, enzyme, polypeptide, oligopeptide, peptide, deoxyribonucleic acid (DNA), ribonucleic acid (RNA), a small organic molecule of less than 900 Da, or a pro-drug of any one of these materials, is a nutritional supplement, or a microbial culture, or a microbial additive, or a vaccine. Further embodiments of the entrapped material are selected from:

a) Proteins, Human growth hormone (hGH), Calcitonin, Insulin, GLP-1 analogues, GLP-1,
b) Peptides, Octreotide,
c) Oral vaccines, Oral cholera vaccine, Mycoplasma hyopneumoniae oral vaccine, Live bacterial cells as attenuated vaccines,
d) Small organic molecules 200<MW<900 g/mol, Desmopressin, Vasopressin, Cyclo-sporine, Ranitidine, Diclofenac, Ketoprofen, Amifostine, Omeprazole, Gemcitabine, Domperidone, Paclitaxel, Cinnarizine, Donepezil, Leucovorin, Raloxifene, Indomethacin, Dextromethorphan, Nizatidine, Peptide Val-Leu-Pro-Val-pro-Arg (VLPVPR), Flurbiprofen, Mebendazole, Thymidine, Zolpidem tartarate, Loratidine, Venlafaxine, Tamsulosin, Urapidil, Prednisolone, Miconazole, Diltiazem, Ambroxol, Captopril, Acyclovir, Cimetidine, Metoprolol, Griseofulvin, Atazanavir, Ibuprofen, Azithromycin, Lercanidipine, Sulfacetamide, Azelastine, Zidovudine, Cloricromene, Oxymatrine, Acarbose, Propranolol, Alfuzosin, Stavudine, Lobenzarit, Genistein, Verapamil, Terbinafine, Lornoxicam, Clotrimazole.

In a still further embodiment the first component and the second component are mixed.

In a further embodiment the first component and the second component are in different layers with the first component being the outer layer and the second component the inner layer around the core.

In a still further embodiment the pair of the first and second component is selected from dietary fibers and enzymes that digest dietary fibers like cellulose and cellulase, pectin and pectinolytic enzymes, hemicellulose and hemicellulase, lignin and lignin degrading enzymes, and fructan and fructan degrading enzymes.

In a further embodiment the pair of the first and second component is selected from structurally ordered cellulose I, such as bacterial cellulose or derivatives thereof and a cellulase, such as cellulase (EC 3.2.1.4) (endocellulase) and cellubiase (EC 3.2.1.21) (beta-glucosidase) and 1,4-beta-cellobiosidase (EC 3.2.1.91) (Exocellulase). Further usable cellulases are Cellulose 1,4-beta-cellobiosidase (reducing end) (EC 3.2.1.176) (exocellulase) as well as cellulase complexes and mixtures thereof.

It is to be understood that the expression "cellulase" as used herein has the meaning understood by the person skilled in the art and covers all cellulases, such as endocellulase, exocellulase, cellulase complex, beta-glucosidase, and mixtures hereof.

In a still further embodiment, the first component is a cellulose and the second component is a cellulase selected from one or more of endocellulases, exocellulases, and beta-glucosidase. Preferably the first component comprises a structurally ordered cellulose I, such as a bacterial cellulose and the second component comprises an endocellulase. Typically, the cellulase, such as the endocellulase, has a cellulose binding domain and is active towards bacterial cellulose.

In a further embodiment the first component is a cellulose and the second component is a cellulase complex.

In a still further embodiment, the second component is enzymatically active and digests the first component in the environment in the intestine of a mammal. Typically, the second component has maximum enzymatic activity in the range pH 3-12, such as from pH 3.5 to 9.5, or from pH 3.0 to 9.0, or from pH 4.0 to 9.0.

In a further embodiment the composition is a tablet or a capsule or other type of solid oral dosage form.

In a further aspect the present invention relates to use of a pair of components for preparing a coating for a solid oral dosage form where the coating is degraded when subjected to a pH change from a lower to a higher pH, wherein the digestion of the first component by the second component is inhibited at the lower pH and the second component digest the first component when subjected to the higher pH.

## Description of the invention

The present invention relates to a solid oral composition for targeted release in an intestine of a mammal. The solid oral composition may be any form known to the skilled person and in particular is a tablet or a capsule, to be administered via the oral route to a mammal. In one embodiment the oral composition is a tablet. In another embodiment the oral composition is a capsule. The mammal can be any mammal, such as a human.

The term "mammal" as used herein means animals, such as without limitation humans, dog, cat, pig, monkey, human apes, rodents. The term "mammal with little or no ability to digest cellulose" means human, dog, cat, pig, monkey, human apes, rodents and other animals with little or no ability to digest cellulose. Mammals, such as cows, cattle, horse, goat, sheep and deer, that natively have a digestion system with ability to digest cellulose polymers do not benefit from the present invention when the coating contains celluloses.

The solid oral composition for targeted release in the intestine of a mammal, such as a human, refers to the design of the solid composition as explained in detail below, which design allows accurate sensing of when the composition reaches the intestine, which is the target, hence targeted release.

The solid composition comprises a core, which core may contain a solution, suspension or be a solid core, provided the core itself does not have any influence on the stability of the coating. The core is surrounded by a coating which defines the outer solid layer, hence the overall composition is referred to as a solid composition. In the core a material is contained, and entrapped, which material is to be released in the intestine upon the coating or part of the coating being dissolved or destroyed. The entrapped material can be any material suitable for release in the intestine, such as without limitation, a drug, a nutrient, a supplement, a microbial organism, a vaccine, a radio transmitter, a device for measuring parameters in the intestine, etc. The coating or a part of the coating is adapted to be digested in the intestine, which means that the coating may consist of different coatings, such as a part to be digested and release the entrapped material and another part which remains intact, or the whole coating is to be digested or perforated in the intestine and release the entrapped material. The intestine comprises different elements, such as the small intestine, large intestine which consists of different elements duodenum, ileum, jejunum, and colon. It is intended that each of these elements can be subject to an embodiment in combination with the aspects and embodiments of the present invention.

Preferably, the coating is adapted to resist breakdown from the environment in a stomach of a mammal, so that no release of the entrapped material takes place in the stomach. The coating may in itself be composed of two or more elements where one enzymatically digests the other in the intestine, but not in the stomach. Thus, the coating prevents the release of the entrapped material in a stomach of a mammal, and/or the coating protects the entrapped material in a stomach of a mammal. When stated that the coating is adapted to release the entrapped material in an intestine of a mammal, it refers to the composition of the coating which composition will be disrupted by enzymatical digestion in the intestine. Such a coating is composed of two or more components, and in accordance with a preferred embodiment of the present invention the coating comprises a pair of components. The pair of components means two components and such components are different in that one must digest the other in order to make this a coating as used in accordance with the invention, wherein the coating or a part of the coating is adapted to be digested in the intestine.

The first component is resistant to the environment in the stomach of a mammal and the second component digest the first component when subjected to the more basic environment of the intestine compared to the more acidic environment of the stomach. In this respect the mammal is preferably selected from animals with little or no ability to digest cellulose, such as a human, a monkey, a pig, a dog, a human ape, a rodent and a cat. In a preferred embodiment the mammal is a human. The reference to first and second is only to indicate that the two components are different but does in no way refer to the way or any order of making the coating. Preferably, the second component digests the first component by having a digesting activity that is inhibited in the environment in the stomach of the mammal. The second component if not inhibited in the environment in the stomach may become a less workable solution although this could be compensated for by adding a further coating covering the solid oral composition of the present invention.

One method of making a coating for use in accordance with the present invention is the preparation of the pair of components. The first component of the coating composition for use in the present invention can for instance be produced by first producing a bacterial cellulose (BC) cuticle by microbial fermentation, secondly purify the BC. This procedure may include an alkaline treatment step. Then the second component, such as a cellulase can be produced by microbial fermentation, chemical synthesis or other means, followed by a purification of this cellulase, for instance by purifying by affinity chromatography. Hereafter, the cellulase is made in a fluid formulation and infused into the BC cuticle (for instance at a pH or at other conditions where the cellulase is inactive). Dry the cuticle to obtain sheets of BC impermeable to molecules of MW > 200 Da containing the cellulase (CX). Embed core (comprising the entrapped material to be released in the intestine) in the BC/CX sheets and seal with an appropriate component. An outline of a coating production process is shown in figure 2.

The entrapped material can be anything which is suitable for release in the intestine, such as compounds for use as medicine.

In a further embodiment the entrapped material is a drug. In a still further embodiment the entrapped material is a nutritional supplement. In a further embodiment the entrapped material is a microbial culture. In a still further embodiment the entrapped material is a microbial additive. In a further embodiment the entrapped material is a vaccine.

Further embodiments of the drug are selected from one or more of a protein, an enzyme, a polypeptide, an oligopeptide, a peptide, a deoxyribonucleic acid (DNA), ribonucleic acid (RNA), a small organic molecule of less than 900 Da, or a pro-drug of any one of these materials, and all of these are considered individual embodiments and may be subject to one or more claims in respect of any one of the aspects and embodiments of the aspects described herein.

Further embodiments of the entrapped material are selected from one or more of:

a) Proteins, Human growth hormone (hGH), Calcitonin, Insulin, GLP-1 analogues, GLP-1,
b) Peptides, Octreotide,
c) Oral vaccines, oral cholera vaccine, *Mycoplasma hyopneumoniae* oral vaccine, live bacterial cells as attenuated vaccines,
d) Small organic molecules 200<MW<900 g/mol, Desmopressin, Vasopressin, Cyclo-sporine, Ranitidine, Diclofenac, Ketoprofen, Amifostine, Omeprazole, Gemcitabine, Domperidone, Paclitaxel, Cinnarizine, Donepezil, Leucovorin, Raloxifene, Indomethacin, Dextromethorphan, Nizatidine, Peptide Val-Leu-Pro-Val-pro-Arg (VLPVPR), Flurbiprofen, Mebendazole, Thymidine, Zolpidem tartarate, Loratidine, Venlafaxine, Tamsulosin, Urapidil, Prednisolone, Miconazole, Diltiazem, Ambroxol, Captopril, Acyclovir, Cimetidine, Metoprolol, Griseofulvin, Atazanavir, Ibuprofen, Azithromycin, Lercanidipine, Sulfacetamide, Azelastine, Zidovudine, Cloricromene, Oxymatrine, Acarbose, Propranolol, Alfuzosin, Stavudine, Loben-zarit, Genistein, Verapamil, Terbinafine, Lomoxicam, Clotrimazole. Each of the compounds or vaccines are considered individual embodiments and may be subject to one or more claims in respect of any one of the aspects and embodiments of the aspects described herein.

The pair of components may be mixed or may be in separate layers. Other ways of making a coating consisting of two components are contemplated by the invention as long as the two components are in contact with each other. For instance, the first component and the second component are mixed. Alternatively, the first component and the second component are in different layers, such as two layers wherein the first component being the outer layer and the second component the inner layer around the core.

In a still further embodiment the pair of the first and second component is dietary fibers and enzymes that digest dietary fibers. In a further embodiment the pair of the first and second component is cellulose and cellulase. In a further embodiment the pair of the first and second component is pectin and pectinolytic enzymes. In a still further embodiment the pair of the first and second component is hemicellulose and hemicellulase. In a further embodiment, the pair of the first and second component is lignin and lignin degrading enzymes. In a still further embodiment the pair of the first and second component is fructan and fructan degrading enzymes.

When the pair of the first and second component is cellulose and cellulase, preferably, the first and second component is selected from structurally ordered cellulose I and a cellulase. The most abundant type of native cellulose found in nature is called cellulose I [6, 7, 8]. The structurally ordered cellulose I is preferably, a bacterial cellulose or derivatives thereof and the cellulase is preferably selected from the group consisting of cellulase (EC 3.2.1.4) (endocellulase), cellubiase (EC 3.2.1.21) (beta-glucosidase) 1,4-beta-cellobiosidase (EC 3.2.1.91) (Exocellulase), Cellulose 1,4-beta-cellobiosidase (reducing end) (EC 3.2.1.176) (exocellulase) as well as cellulase complexes and mixtures thereof.

The term "structurally ordered cellulose I" or "native cellulose" as used interchangeable herein refers to an unbranched polymer containing any number of eight or more D-glucose units, linked by β-1,4 glycosidic bonds.

In a still further embodiment the second component is enzymatically active and digests the first component in the environment in the intestine of a mammal. In particular, the second component has maximum enzymatic activity in the range pH 3-12, such as pH 3.5 to 9.5, or 3.0 to 9.0, or 4.0 to 9.0.

Furthermore, the present invention relates to a solid oral composition for targeted release in an intestine of a mammal comprising a core and a coating completely surrounding the core, wherein the core comprises an entrapped material to be released in the intestine, and the coating or a part of the coating is adapted to be digested in the intestine, for use as a medicinal product. Each of the above described embodiments in relation to the first aspect also applies to this particular use aspect.

In a further aspect the present invention relates to use of a pair of components for preparing a coating for a solid oral dosage form where the coating is degraded when subjected to a pH change from a lower to a higher pH, wherein the digestion of the first component by the second component is inhibited at the lower pH and the second component digest the first component when subjected to the higher pH. The coating may be used to surround the solid oral dosage form or cover a part of the dosage form as the case may be. Typically, the solid oral dosage form is a tablet, a capsule or granulates. Typically, the lower pH is in the range 1.0 - 4.5 and the higher pH is in the range 4.5- 8.5 in the intestine. The creation of the coating according to this further aspect which coating can be stored for later use is also contemplated by this aspect of the invention. The coating can be used in setting up test systems to verify a suitable pair of components and amounts and ratios to be used.

The above embodiments should be seen as referring to any one of the aspects (such as 'composition, 'pharmaceutical composition', 'composition for use as a medicinal product, or 'compound for use in a method') described herein as well as any one of the embodiments described herein unless it is specified that an embodiment relates to a certain aspect or aspects of the present invention.

All headings and sub-headings are used herein for convenience only and should not be construed as limiting the invention in any way.

The terms "a" and "an" and "the" and similar referents as used in the context of describing the invention are to be construed to cover both the singular and the plural, unless otherwise indicated herein or clearly contradicted by context.

The term "and/or" as used herein is intended to mean both alternatives as well as each of the alternatives individually. For instance, expression "xxx and/or yyy" means "the xxx and yyy; the xxx; or the yyy", all three alternatives are subject to individual embodiments.

Recitation of ranges of values herein are merely intended to serve as a shorthand method of referring individually to each separate value falling within the range, unless other-wise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

All methods described herein can be performed in any suitable order unless other-wise indicated herein or otherwise clearly contradicted by context.

The use of any and all examples, or exemplary language (e.g., "such as") provided herein, is intended merely to better illuminate the invention and does not pose a limitation on the scope of the invention unless otherwise indicated.

The citation and incorporation of patent documents herein is done for convenience only and does not reflect any view of the validity, patentability and/or enforceability of such patent documents.

The description herein of any aspect or embodiment of the invention using terms such as "comprising", "having", "including" or "containing" with reference to an element or elements is intended to provide support for a similar aspect or embodiment of the invention that "consists of', "consists essentially of', or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

The present invention is further illustrated by the following examples that, however, are not to be construed as limiting the scope of protection. The features disclosed in the foregoing description and in the following examples may, both separately and in any combination thereof, be material for realizing the invention in diverse forms thereof.

**Experimental procedures**

**Abbreviations**

| BC | Bacterial cellulose |
|---|---|
| CCH | Cumulated cellulose hydrolysis |
| mma | Minutes of maximal activity |
| %ma | Percentage of maximal activity |
| pHd | pH duodenum |
| pHs | pH stomach |
| SRB | Sulforrodamin B |

(continued)

| PBS | Phosphate buffered saline |
|---|---|
| HCl | Hydrochloride |
| OD | Optical density |
| h | Hours |
| nm | Nanometer |
| DNS | Dinitrosalicylic |
| MW | Molecular weight |
| St. Dev. | Standard deviation |

## Methods and results

Physiological parameters were extracted for gastro intestinal pH and passage times in humans from literature. From these parameters, a semi-physiological model of the pH in the GI tract was synthesized and validated against literature data. Further, we extracted biochemical parameters for cellulases characterized by having activity maxima around neutral pH and strongly inhibited activity at acidic pH from the Brenda bioinformatics database [9] and from literature [10, 11, 12]. From these data a semi-biochemical model of the pH dependency of cellulase activity was synthesized and validated against literature data. By combining these two models a semi-physiological model of cumulated cellulase activity was synthesized and used to perform simulations of cellulase passing through the human GI tract.

Results: The two synthesized models predicted the literature data well. Simulations showed that the cellulase activity is strongly inhibited as the cellulase passes through the stomach and that the cellulase becomes active when entering the small intestine. Further, simulations showed only a small amount of cumulated activity (cumulated cellulose breakdown) takes place during the passage of the stomach compared to the cumulated activity (cumulated cellulose breakdown) that takes place during the passage of the small intestine, even at physiologically highly prolonged stomach passage times and physiologically very short small intestine passage time. This shows there is a robust design space for a cellulose-cellulase based coating which protects the entrapped material during the passage of the stomach and which perforates in the intestine.

The entero coating presented here is a method which robustly protects an entrapped material during the passage of the stomach and which robustly releases the material in the intestine through a self-perforating mechanism.

## Results

Cellulases with a maximal activity in the pH range 5.5 - 8.0 and with activity at most 25 % of maximal activity (%ma) in acidic environments (pH < 4.0) were identified in the Brenda bioinformatics database and in literature [9, 10, 11, 12] and biochemical characterization data from these cellulases were used to synthesize a model ($A_1$(pH)) predicting the relative cellulase activity as a function of pH.

$$A_1(pH) = 100 * exp\left(-\left(\frac{|pH-6.5|}{2}\right)^3\right) \%ma$$

The synthesised activity function $A_1$(pH) models a cellulase with maximal activity at pH 6.5.

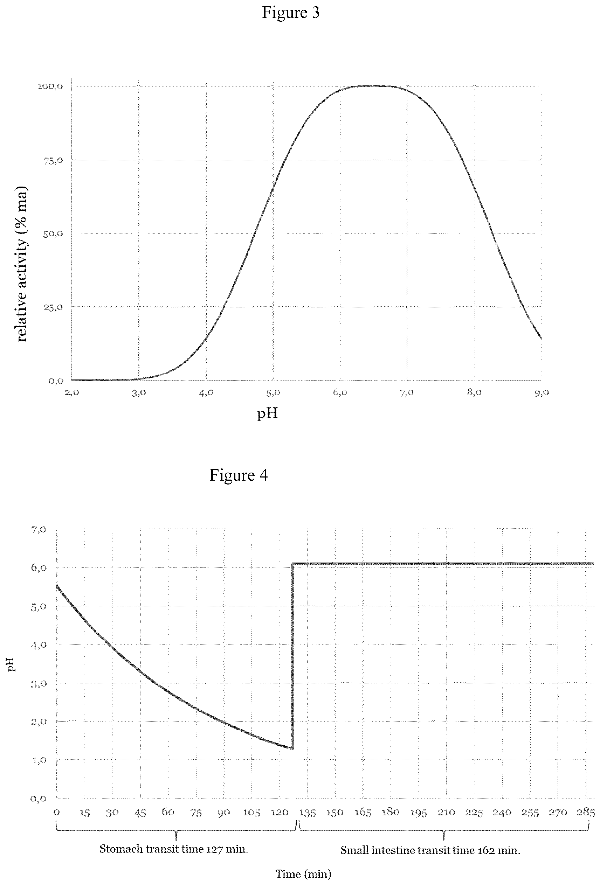

A graphic representation of the relative activity as a function of pH is shown in figure 3. The model was validated against literature data by overlaying the synthesized $A_1$(pH) activity function with the activity data from cellulases identified in the literature [10, 11, 12].

A model for pH(t) in the human gastro intestinal system was synthesized by combining physiological data from the literature [1, 2].

The model for the variation of pH in the stomach (*pHs(t)*) of healthy young men and women with time after meal ingression was extracted from the study [1].

$$pH_S(t) = 0.5 + \exp\left(-0.016 * t + 1.7\right)$$

The model with constant pH = 6.1 for the pH in the small intestine is based on the study [2]. Denoting by $T_S$ the stomach transit time and by $T_I$ the intestinal transit time we obtain a model pH(t) predicting the human gastro intestinal pH environment surrounding a capsule/ an entrapped material as it passes through the intestine. Figure 1 shows a graphic representation of the synthesized model $A_1$(pH) predicting the relative cellulase activity as a function of pH.

$$pH(t) = \begin{cases} 0.5 + \exp(-0.016 * t + 1.7), & 0 \leq t \leq T_S, \\ 6.1, & T_S \leq t \leq T_S + T_I. \end{cases}$$

The model *pH(t)* was plotted on top of human stomach and intestinal physiological data and predicts well the data on gastro intestinal pH in healthy humans found in the literature [1, 2]. Figure 4 shows the graphic representation of the model predicting the gastro intestinal pH(t) in humans with a stomach transition time Ts of 127 min and a short small intestine transit time $T_I$ of 162 min.

By combining the model ($A_1$(pH)) predicting the relative cellulase activity as a function of pH with the model predicting the gastro intestinal pH(t) in humans, a model of relative cellulase activity in the GI tract was obtained by composing the two functions:

$$A_1(pH(t))$$

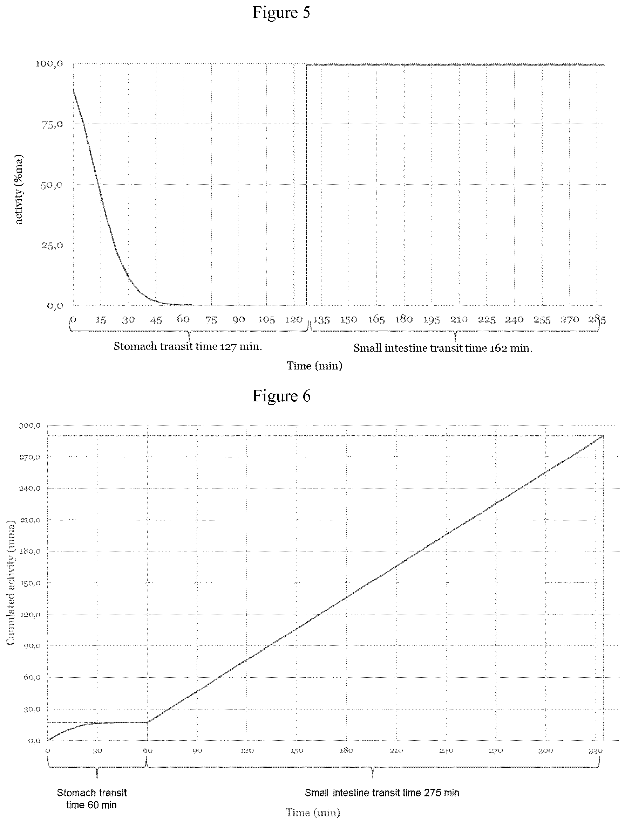

Where $A_1$(*pH*) is the cellulase activity function and *pH*(*t*) is the human gastro intestinal pH function. Figure 5 shows a graphic representation of the model of relative cellulase activity in the gastro intestinal tract. The relative cellulase activity in the GI tract with a stomach transition time $T_S$ of 127 min and a small intestine transit time $T_I$ of 162 min is shown.

A model predicting the cumulated activity in the self-perforating material, where the first component is cellulose, and the second component is a cellulase, is obtained by integrating the relative activity function $A_1$(*pH*(*t*))/100 with respect to time. A graphic representation of the cumulated activity in the self-perforating material with a median stomach transition time $T_S$ of 60 min and a median small intestine transit time $T_I$ of 275 min is shown in figure 6.

For the calculations of cumulated activity, a relative unit minutes of maximal activity (mma) is used. The mma unit describes the cumulated cellulase activity as equivalents of reaction minutes when the reaction is performed at optimal conditions. e.g. at pH 6.5, the optimal reaction pH, in the case of the model cellulase described in $A_1$(pH).

- The basic unit 1 mma is the enzymatic cellulase turnover of β-1, 4-glycosidic bonds at maximal enzymatic activity for one minute.
- The mma unit is related to a specific number of active sites e.g. amount of cellulase and a specific number of 1-4 glycosidic bounds, e.g. amount of cellulose
- The mma unit is specific to individual cellulases and to individual cellulose substrates

The mma unit can be used to compare the cumulated cellulase activity under different reaction conditions, for example reaction time, temperature, pH, cofactor concentration and inhibitor concentrations. The mma unit is convenient for product design and may be used as a production specification to design a material which self-perforate within a certain range of cumulated cellulase activity.

**Example 1**

The model of the performance of the self-perforating material in the gastro-intestinal system $A_1$(*pH*(*t*))/100 was used to study the activity cumulating in the self-perforating material when passaging the human GI tract under different physiological conditions. Five studies were made to explore, under different stomach emptying times and small intestine transition times, the properties of the self-perforating material, where the first component is cellulose, and the second component is a cellulase with the activity function $A_1$(pH).

*A stomach transition time of 60 min and a small intestine transit time of 275 min*

A typical median stomach emptying time was found in the literature to be 60 min [3,4,5] and a typical median intestinal transition time was found to be 275 min in the literature [3,4,5]. A graphic representation of the cumulated cellulose hydrolysis predicted by the model using these stomach-emptying and small intestine transition times is shown in figure 6. Figure 2 shows the model predicting the cumulated activity in the self-perforating material in the gastro-intestinal system. Input parameters: a stomach transition time Ts of 60 min and a small intestine transit time $T_I$ of 275 min.

The model of the cumulated activity in the GI tract shows that a self-perforating material designed to withstand a cumulated activity of more than 17 mma will protect the entrapped material from the environment in the stomach with a stomach emptying time of up to 60 min. In addition, a self-perforating material designed to be digested with a cumulated activity of less than 290 mma will release the entrapped material in the human small intestine given a small intestine transit time of 275 min. Thus, a self-perforating material designed to become digested by a cumulated activity in the range 17 to 290 mma will release the entrapped material with typical median stomach and small intestinal transit times.

*A stomach transition time of 5 min and a small intestine transit time of 162 min*

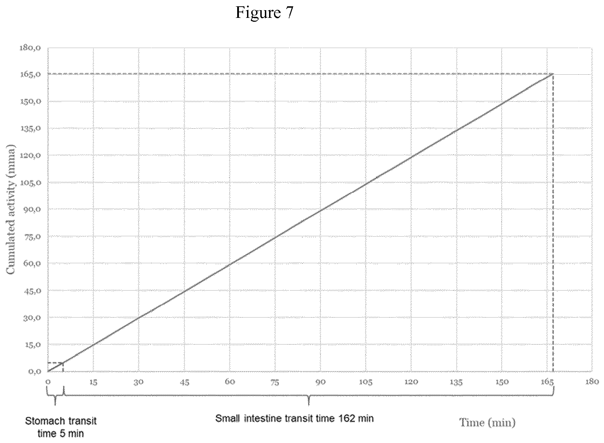

A typical short stomach emptying time was found in the literature to be 5 min [3,4,5] and a typical short intestinal transition time was found to be 162 min in the literature [3,4,5]. A graphic representation of the cumulated cellulose hydrolysis predicted by the model using these stomach-emptying and small intestine transition times is shown in figure 7. Figure 3 shows the model predicting cumulated activity in the self-perforating material in the gastro-intestinal system. Input parameters: a stomach transition time Ts of 5 min and a small intestine transit time $T_l$ of 162 min.

The model of the cumulated activity in the GI tract shows that a self-perforating material designed to withstand a cumulated activity of more than 5 mma will protect the entrapped material from the environment in the human stomach with a stomach emptying time of up to 5 min. In addition, when designed to be digested with a cumulated activity of less than 165 mma it will release the entrapped material in the human small intestine given a small intestine transit time of 162 min. Thus, a self-perforating material designed to become digested by a cumulated activity in the range 5 to 165 mma will release the entrapped material with typical short stomach and small intestinal transit times.

*A stomach transition time of 337 min and a small intestine transit time of 162 min*

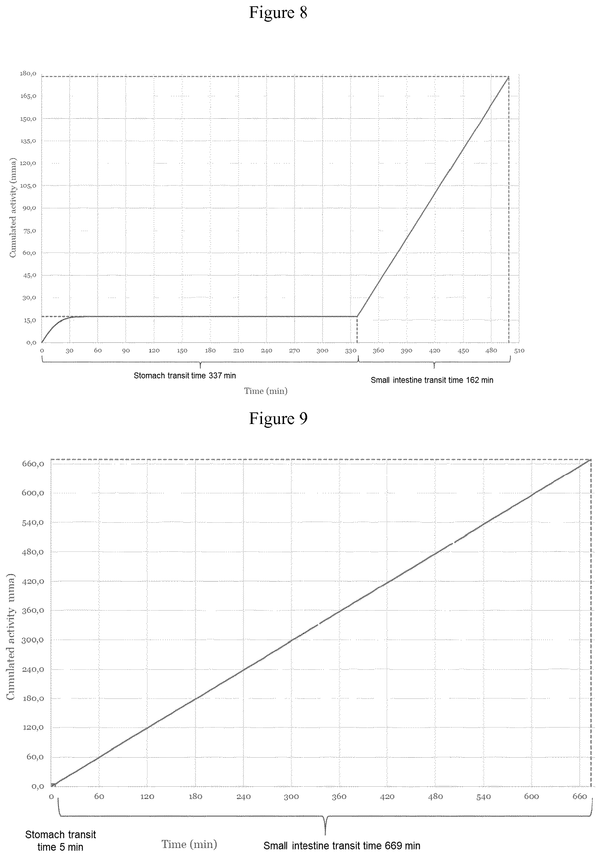

A typical long stomach emptying time was found in the literature to be 337 min [3,4,5] and a typical short intestinal transition time was found to be 162 min in the literature [3,4,5]. A graphic representation of the cumulated cellulose hydrolysis predicted by the model using these stomach-emptying and small intestine transition times is shown in figure 8. Figure 8 shows the model predicting the cumulated activity in the self-perforating material in the gastro-intestinal system using input parameters: stomach transition time Ts of 337 min; small intestine transit time $T_l$ of 162 min.

The model of the cumulated activity in the GI tract shows that a self-perforating material designed to withstand a cumulated activity of more than 17 mma will protect the entrapped material from the environment in the human stomach with a stomach emptying time of up to 337 min. In addition, if designed to be digested with a cumulated activity of less than 178 mma the entrapped material will be released in the human small intestine given a small intestine transit time of 162 min. Thus, a self-perforating material designed to become digested by a cumulated activity in the range 17 to 178 mma will release the entrapped material with typical long stomach and short small intestinal transit times.

*A stomach transition time of 5 min and a small intestine transit time of 669 min*

A typical short stomach emptying time was found in the literature to be 5 min [3,4,5] and a typical long intestinal transition time was found to be 669 min in the literature [3,4,5]. A graphic representation of the cumulated cellulose hydrolysis predicted by the model using these stomach-emptying and small intestine transition times is shown in figure 9. Figure 9 shows the model predicting the cumulated activity in the self-perforating material in the gastro-intestinal system. Input parameters: a stomach transition time Ts of 5 min and a small intestine transit time $T_l$ of 669 min.

The model of the cumulated activity in the GI tract shows that a self-perforating material designed to withstand a cumulated activity of more than 5 mma will protect the entrapped material from the environment in the human stomach with a stomach emptying time of up to 5 min. In addition, a self-perforating material designed to be digested with a cumulated activity of less than 668 mma will release the entrapped material in the human small intestine given a small intestine transit time of 669 min. Thus, a self-perforating material designed to become digested by a cumulated activity in the range 5 to 668 mma will release the entrapped material with typical long stomach and short small intestinal transit times.

*A stomach transition time of 337 min and a small intestine transit time of 669 min*

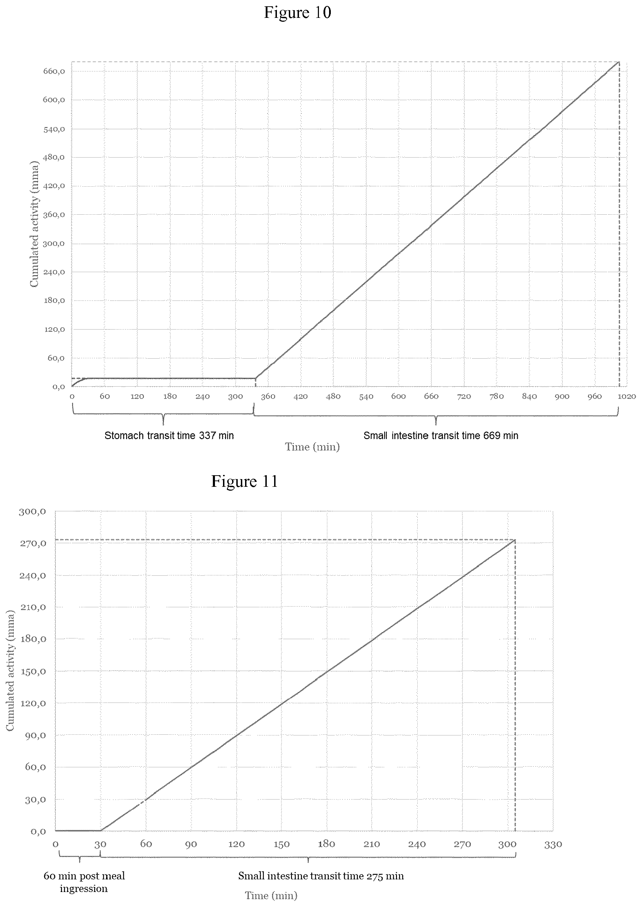

A typical long stomach emptying time was found in the literature to be 337 min [3,4,5] and a typical long intestinal transition time was found to be 669 min in the literature [3,4,5]. A graphic representation of the cumulated cellulose hydrolysis predicted by the model using these stomach-emptying and small intestine transition times is shown in figure 10. Figure 10 shows the model predicting the cumulated activity in the self-perforating material in the gastro-intestinal system. Input parameters: a stomach transition time Ts of 337 min and a small intestine transit time $T_l$ of 669 min.

The model of the cumulated activity in the GI tract shows that a self-perforating material designed to withstand a cumulated activity of more than 17 mma will protect the entrapped material from the environment in the human stomach with a stomach emptying time of up to 337 min. In addition, a self-perforating material designed to be digested with a cumulated activity of less than 681 mma will release the entrapped material in the human small intestine given a small intestine transit time of 669 min. Thus, a self-perforating material designed to become digested by a cumulated activity in the range 17 to 681 mma will release the entrapped material with typical long stomach and long small intestinal transit times.

In the five studies in example 1 all combinations of typical short and long stomach emptying times and typical short and long intestinal transition times were studied. The self-perforating material, where the first component is cellulose, and the second component is a cellulase with the activity function $A_1$(pH), can be designed to protect the entrapped material from the environment in the human stomach, and release the entrapped material in the human small intestine in any of the above considered cases if the material is designed with a cumulated activity specification in the window 17 mma to 165 mma.

**Example 2**

The model predicting the performance of the self-perforating material in the gastro-intestinal system $A_1$(pH(t))/100 was modified to study the performance of the self-perforating material in the human GI tract when the coating is given a time $T_{PM}$ post meal ingression. The formula for the pH variation then becomes:

$$pH(t) = \begin{cases} 0.5 + \exp\left(-0.016 * (T_{PM} + t) + 1.7\right), & 0 \leq t \leq T_S, \\ 6.1, & T_S \leq t \leq T_S + T_I. \end{cases}$$

The obtained model was used to study the cumulated activity when the coating is given at $T_{PM}$ = 60 min post meal ingression and considering a stomach transit time $T_S$ of 30 min. A graphic representation is shown in figure 11. Figure 4 shows the cumulated activity in the self-perforating material in the human gastro intestinal tract when the material is given at $T_{PM}$ = 60 min post meal ingression and considering a stomach transit time $T_S$ of 30 min and a small intestine transit time $T_S$ of 275 min.

When the coating is given 60 min post meal ingression the cellulase activity is very low because of the acidic environment. This is reflected in the predicted cumulated activity of 0 mma in the stomach. When the coating material enters the small intestine the cellulase activity increases, initiating the breakdown of the coating, and supporting the release of the entrapped material. By ingression of the coating at a delayed time following a meal, the cumulated activity in the stomach is decreased significantly compared to when the coating is ingressed together with a meal. Thus, the design space for the coating is increased significantly.

**Example 3**

The effects on the cumulated activity in the gastro intestinal tract of using cellulase enzymes with other pH optima was studied.

The model predicting the performance of the self-perforating material in the gastro-intestinal system was modified to $A_2$(pH(t))/100 to predict the performance in the human GI tract of a cellulose-cellulase self-perforating material, when the cellulase has maximal activity at $pH_{OPT}$. $A_2$(pH) models a cellulase enzyme with characteristics similar to the cellulase properties described in model $A_1$(pH), however, with an enzymatic optimum at $pH_{OPT}$.

$$A_2(pH) = 100 * exp\left(-\left(\frac{|pH - pH_{OPT}|}{2}\right)^3\right) \%ma$$

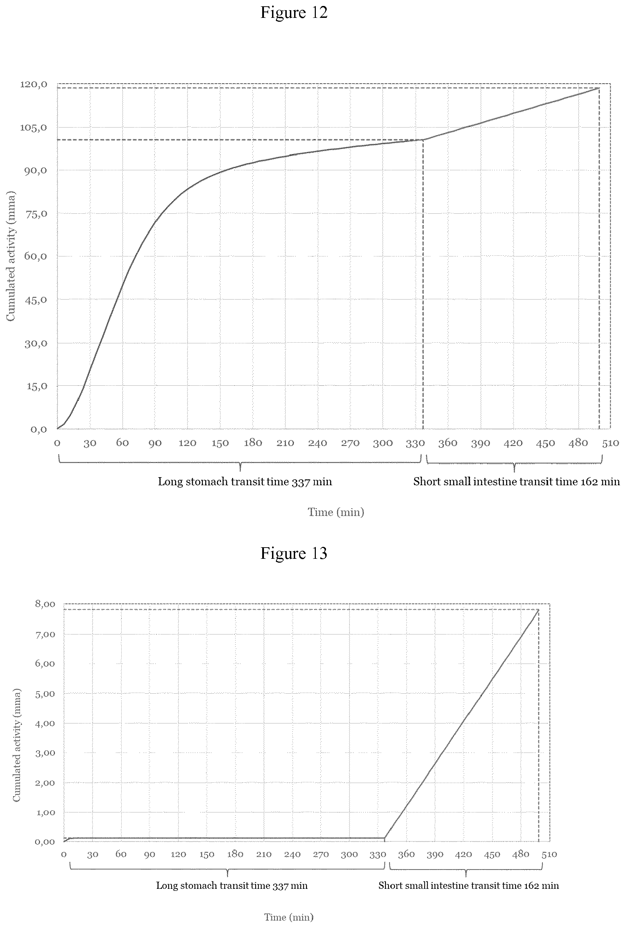

$A_2$(pH) was used to study the cumulated activity of an enzyme with maximal activity at $pH_{OPT}$ equal to 3.5. A graphic representation is shown in figure 12. Figure 5 shows the graphic representation of the cumulated activity of an enzyme with maximal activity at $pH_{OPT}$ = 3.5 described in function $A_2$(pH(t))/100.

$A_2$(pH) was used to study the cumulated activity of an enzyme with the maximal activity at $pH_{OPT}$ equal to 9.0. A graphic representation is shown in figure 13. Figure 6 shows the graphic representation of the cumulated activity of an enzyme with maximal activity at $pH_{OPT}$ = 9.0 described by function $A_2$(pH(t))/100.

In a composition with a cellulase with maximal activity at pH 3.5 the cumulated activity in the stomach levels out at almost zero at prolonged stomach transit times (figure 12). A composition with an enzyme with maximal activity at pH 3.5 can protect the entrapped material in the stomach when the coating material is designed to release the entrapped material in the range 100 mma to 118 mma. However, such a composition with an enzyme with maximal activity at pH 3.5 may require that the coating is given post meal ingression to obtain a robust design space for release of the entrapped material in the small intestine.

A composition with an enzyme with maximal activity at pH 9.0 can protect the entrapped material in the stomach when the coating material is designed to release the entrapped material in the range 0.1 mma to 7.8 mma. A very robust design space for releasing the entrapped material in the intestine can be obtained with such a composition.

A composition with an enzyme with maximal activity above pH 9.0 will only improve the ability of the material to protect the entrapped material in the stomach and release the entrapped material in the intestine.

A composition with a cellulase enzyme with an enzymatic optimum in the range from at least pH 3.0 to 12.0, such as from 3.5 to 9.0, can serve as the second component in the self-perforating material, and support the protection of the entrapped material in the stomach and a release in the small intestine.

**Example 4**

The purpose of this experiment was to determine the effect of the thickness (measured as specific mass mg/cm$^2$) of a BC membrane and pH on the diffusion of a model compound through the membrane.

Wet BC membrane from cultures of *Komagataebacter xylinus* were compressed to remove water and then freeze-dried (3 days, -99 °C; 0.049 mbar). Membranes with a mass per area of 3.3 mg/cm$^2$ and 13.9 mg/cm$^2$ (thickness) were obtained. Sulforrodamin B (SRB) (MW 558.7 g/mol) was used as model compound. The diffusion over time of SRB through the BC membranes were investigated in a diffusion cell at pH 2 and pH 6.5. A diffusion cell consisting of a donor chamber where the model compound is placed at the beginning of the study, and a receptor chamber into which the model compound may migrate. The membrane to be studied is placed between the two chambers.

The donor chamber was filled with a 2 mg/mL SRB solution in 0,01 M HCl (pH 2) or a PBS (pH 6.5). The membrane to be studied was placed on top of the donor chamber. The study was initiated when the receptor chamber was filled with 0,01 M HCl (pH 2) or PBS (pH 6.5) as corresponding to the pH in the donor chamber. Samples were taken from the receptor chamber after 0 h, 1 h, 1.5 h, 2 h, 4 h and 6 h and the concentration of SRB was measured at OD 565 nm. The results are shown in table 1 and table 2.

Table 1. The concentration of the SRB model compound in the receptor chamber (μg/ml) in a time cause experiment at pH 2.

| Diffusion time (h) | SRB (μg/ml) in the receptor chamber at pH 2 | | | |
|---|---|---|---|---|
| | 3.3 mg/cm$^2$ membrane | 3.3 mg/cm$^2$ membrane St. Dev. | 13.9 mg/cm$^2$ membrane | 13.9 mg/cm$^2$ membrane St. Dev. |
| 0.0 | 0.2 | 0.0 | 0.0 | 0.1 |
| 1.0 | 29.6 | 8.1 | 2.0 | 1.6 |
| 1.5 | 46.3 | 12.9 | 2.9 | 2.4 |
| 2.0 | 66.8 | 17.3 | 4.6 | 3.7 |
| 4.0 | 155.6 | 28.8 | 12.4 | 8.7 |
| 6.0 | 252.5 | 56.7 | 21.5 | 11.9 |

Table 2. The concentration of the SRB model compound in the receptor chamber (μg/ml) in a time cause experiment at pH 6.5.

| Diffusion time (h) | SRB (μg/ml) in the receptor chamber at pH 6.5 | | | |
|---|---|---|---|---|
| | 3.3 mg/cm$^2$ membrane | 3.3 mg/cm$^2$ membrane St. Dev. | 13.9 mg/cm$^2$ membrane | 13.9 mg/cm$^2$ membrane St. Dev. |
| 0.0 | 0.1 | 0.1 | 0.2 | 0.2 |
| 1.0 | 19.8 | 8.5 | 2.5 | 1.2 |
| 1.5 | 31.3 | 13.5 | 4.9 | 2.5 |
| 2.0 | 46.3 | 16.5 | 7.9 | 4.5 |
| 4.0 | 97.5 | 15.1 | 23.3 | 11.6 |

(continued)

| | SRB ($\mu$g/ml) in the receptor chamber at pH 6.5 | | | |
|---|---|---|---|---|
| Diffusion time (h) | 3.3 mg/cm$^2$ membrane | 3.3 mg/cm$^2$ membrane St. Dev. | 13.9 mg/cm$^2$ membrane | 13.9 mg/cm$^2$ membrane St. Dev. |
| 6.0 | 142.3 | 16.2 | 39.2 | 16.7 |

The results in table 1 show that the SRB concentration in the receptor chamber is 66.8 $\mu$g/ml after 2 hours when it diffuses through a BC membrane of 3.3 mg/cm$^2$ at pH 2 and 4.6 $\mu$g/ml after 2 hours when it diffuses through a BC membrane of 13.9 mg/cm$^2$ at pH 2. Equilibrium of the diffusion is reached when the concentration of SRB in the receptor chamber is equal to the concentration of SRB in the donor chamber. The diffusion cell used in this study exhibits diffusion equilibrium around an SRB concentration of 1100 $\mu$g/ml. The concentrations 66.8 $\mu$g/ml and 4.6 $\mu$g/ml attained after 2 hours at pH 2 corresponds to around 6 % (membrane 3.3 mg/cm$^2$) and 0.5 % (membrane 13.9 mg/cm$^2$) of maximal SRB diffusion. 2 hours at pH 2 correspond to a typical maximal stomach passage time and a typical pH in the stomach. It can be concluded that bacterial cellulose can retain and protect an entrapped material during a stomach passage. The results in table 2 show that the BC membrane also retains and protect the entrapped material at pH 6.5 (corresponding to the pH in the intestine) and therefore requires a second component to speed up the release of the entrapped material. Diffusion through the membrane is dependent on the thickness of the membrane.

**Example 5**

The purpose of this experiment was to determine the effect of pH on the activity of a cellulase complex introduced into a bacterial cellulose membrane.

Wet BC membrane was obtained from a culture of *Komagataebacter xylinus* and compressed to remove water. Celluclast 1.5L (Novozymes, DK) cellulase complex solution from *T. reesei* (100 $\mu$l) was added to BC membranes and incubated to allow for the adsorption of the enzymes onto the BC fibers (15 min at 0°C). The cellulase complex infused membranes were freeze-dried (3 days, -99 C; 0.049 mbar). In preparations without infused cellulase the specific weight of the BC membrane was 3.1 mg/cm$^2$.

The digestion activity of the cellulase complex infused into BC membranes at pH 2 and pH 6.5 was investigated. The study was initiated by incubating the membranes at pH 2 and 6.5 (6.7 ml of 0.01 M HCl (pH 2) or PBS (pH 6.5) respectively). Samples were taken after 0h, 1h, 1.5h, 2h, 4h and 6h of cellulose digestion and analyzed for reducing sugars by the DNS method of Miller [13]. The results are shown in table 3.

Table 3. Reducing sugars released from a BC membrane by cellulase complex digestion at pH 2 and pH 6.5

| Digestion time (h) | Cellulase complex activity at pH2. Reducing sugars produced (mM) | St. Dev. Reducing sugars produced (mM) | Cellulase complex activity at pH 6.5 Reducing sugars produced (mM) | St. Dev. Reducing sugars produced (mM) |
|---|---|---|---|---|
| 0.0 | -0.03 | 0.01 | -0.03 | 0.01 |
| 1.0 | 0.49 | 0.05 | 0.96 | 0.04 |
| 1.5 | 0.51 | 0.04 | 1.42 | 0.06 |
| 2.0 | 0.53 | 0.04 | 1.80 | 0.03 |
| 4.0 | 0.53 | 0.08 | 2.29 | 0.13 |
| 6.0 | 0.55 | 0.04 | 2.40 | 0.13 |

The results show that a cellulase complex which is introduced into a cellulose membrane, freeze dried and then rehydrated can be brought to digest the cellulose membrane. This demonstrates the validity of the two-component coating production process shown in figure 2. At pH 2 corresponding to the environment in the stomach, the cellulase activity and therefore the cellulose digestion is inhibited and stops after 1 h. At pH 6.5, corresponding to the environment in the intestine, the BC membrane is digested.

**Example 6**

The purpose of this experiment was to determine the effect of pH on the activity of a combination of cellulases introduced into a bacterial cellulose membrane.

Wet BC membrane was obtained from a culture of *Komagataebacter xylinus* and compressed to remove water. An 8:8:3:6 combination of cellulases was prepared from: 2 endo cellulases from *Thermobifida fusca* and *Clostridium cellulolyticum* (EC: 3.2.1.4 from family 6A and 9G respectively) and 2 exocellulases from *Podospora anserina* and *Thermobifida fusca* (EC: 3.2.1.91 family 6A and EC: 3.2.1.176 family 48A). The enzymes have activity optimum in the range pH 5.0 to 9.0, a cellulose binding domain and have crystalline cellulose as substrate. All enzymes were produced by heterologous expression followed by purification (NZYTech, Portugal).

The cellulase combination solution (415 $\mu$l) was added to compressed BC membranes and incubated to adsorb the enzymes to the BC fibers. (15 min at 0°C). The BC membranes were freeze-dried (3 days, -99 C; 0.049 mbar). In preparations without infused cellulase the specific weight of the BC membrane was 7,2 mg/cm$^2$.

The activity study was initiated by incubating the cellulase-combination infused freeze-dried BC membranes at pH 2 and 6.5 (6.7 ml of 0.01 M HCl (pH 2) or PBS (pH6.5) respectively). Samples were taken after 0h, 1h, 1.5h, 2h, 4h and 6h of digestion and the production of reducing sugars was measured by the method of Miller [13]. The results are shown in table 4.

Table 4. Digestion of BC membranes by a cellulase-combination at pH 2 and pH 6.5.

| Digestion time (h) | Cellulase combination activity pH 2 Reducing sugars produced (mM) | St. Dev. Reducing sugars produced (mM) | Cellulase combination activity pH 6.5 Reducing sugars produced (mM) | St. Dev. Reducing sugars produced (mM) |
|---|---|---|---|---|
| 0 | -0.02 | 0.07 | 0.16 | 0.17 |
| 1.0 | -0.02 | 0.05 | 0.13 | 0.14 |
| 1.5 | 0.00 | 0.01 | 0.08 | 0.02 |
| 2.0 | -0.03 | 0.00 | 0.11 | 0.05 |
| 4.0 | -0.01 | 0.04 | 0.44 | 0.03 |
| 6.0 | -0.05 | 0.01 | 0.70 | 0.06 |

The results show that a cellulase combination which is introduced into a cellulose membrane, freeze dried and then rehydrated can be brought to digest the BC cellulose membrane. This further demonstrates the validity of the two-component coating production process shown in figure 2. At pH 2, corresponding to the environment in the stomach, the activity is strongly inhibited, and no activity can be detected. At pH 6.5, corresponding to the environment in the intestine, the BC membrane is digested.

**Example 7**

The purpose of this experiment was to show the diffusion of a model compound through a BC membrane (component 1) digested by an introduced cellulase (component 2)

Wet BC membrane was obtained from a culture of *Komagataebacter xylinus* and compressed to remove water. Celluclast 1.5L (Novozymes, DK) cellulase complex solution from *T. reesei* (100 $\mu$l) was added to BC membranes and incubated to allow for the adsorption of the enzymes onto the BC fibers (15 min at 0°C). The cellulase complex infused BC membranes were freeze-dried (3 days, -99 C; 0.049 mbar). In preparations without infused cellulase the specific weight of the BC membrane was 7.3 mg/cm$^2$. Sulforrodamin B (SRB) (MW 558.7 g/mol) was used as model compound. The diffusion over time of SRB through the BC membranes were investigated in a diffusion cell at pH 2 and pH 6.5. The donor chamber of the diffusion cell was filled with 2,9 mL of 2 mg/mL SRB solution at pH 2 or pH 6.5. The membrane to be studied was placed on top of the donor chamber. The study was initiated when the receptor chamber was filled with 0,01 M HCl (pH 2) or PBS (pH 6.5) as corresponding to the pH in the donor chamber. The diffusion cell was placed at 50 °C. Samples were taken from the receptor chamber at 0h, 1h, 2h, 3h, 4h and 6h and the concentration of SRB was measured at OD 565 nm. The results are shown in table 5.

# EP 3 836 905 B1

Table 5. Diffusion of a model compound through a BC membrane which is, at the same time, digested by a cellulase complex that was previously introduced into the membrane.

| | Concentration of SRB ($\mu$g/ml) in receptor chamber | | | |
|---|---|---|---|---|
| Activity and diffusion time (h) | Activity and diffusion at pH 2 SRB ($\mu$g/ml) | St. Dev. SRB ($\mu$g/ml) pH 2 | Activity and diffusion at pH 6.5 SRB ($\mu$g/ml) | St. Dev. SRB ($\mu$g/ml) pH 6.5 |
| 0.0 | 0.2 | 0.0 | 0.0 | 0.0 |
| 1.0 | 0.4 | 0.1 | 22.5 | 3.1 |
| 2.0 | 0.9 | 0.2 | 62.7 | 4.0 |
| 3.0 | 6.2 | 3.9 | 118.3 | 8.1 |
| 4.0 | 12.7 | 6.7 | 211.3 | 35.6 |
| 6.0 | 30.1 | 10.7 | 588.0 | 201.6 |

The results show that diffusion of a model compound through a BC membrane digested by an infused cellulase complex is constant at a lower rate at pH 2 and is at a higher rate at pH 6.5. The results exemplify the use of a pair of components for preparing a coating where 1) the coating is digested, when subjected to the higher pH but not at the lower pH, 2) the first component (exemplified here by bacterial cellulose) is indigestible by the natural digestion in the gastro-intestinal system and 3) the second component (exemplified here by a cellulase) digest the first component when subjected to the higher pH in the intestine, but not when subjected to the lower pH in the stomach. Thus, the BC membrane coating prevents the release of the model compound under typical conditions found in the stomach (pH 2) and promotes the release of the model compound under typical conditions found in the intestine of a mammal (pH 6.5).

## Conclusion

Based on the studies presented the described coating material allows protection of the entrapped material in the stomach of a mammal, such as a human, and allows for release in the intestine. Together example 1 to 3 show that a high degree of robustness of design space can be obtained, allowing for a wide range of coating specifications. Together example 4 to 7 shows the technical feasibility of the described coating material.

## References

[1] Dressman JB, Berardi RR, Dermentzoglou LC, Russell TL, Jarvenpaa KM. Upper Gastrointestinal (GI) pH in Young, Healthy Men and Women. Pharm Res. 1990; 7:756-761.

[2] Clarysse S, Tack J, Lammert F, Duchateau G, Reppas C, Augustijns P. Postprandial Evolution in Composition and Characteristics of Human Duodenal Fluids in Different Nutritional States. J. Pharm Sci. 2009; 98:1177-1192.

[3] Zarate N, Mohammed SD, O'Shaughnessy E, Newell M, Yazaki E, Williams NS, Lunniss PJ, Semler JR, Scott SM. Am J Physiol - Gastroint and Liver Physiol 2010; 299 (6): G1276-G1286. Accurate localization of a fall in pH within the ileocecal region: Validation using a dual-scintigraphic technique.

[4] Worsøe et al. Gastric transit and small intestinal transit time and motility assessed by a magnet tracking system BMC Gastroenterology 2011, 11:145

[5] Assessment of regional gut transit times in healthy controls and patients with gastroparesis using wireless motility technology. I. Sarosiek et al. Aliment Pharmacol Ther. 2010 January 15; 31(2): 313-322

[6] Occurrence of cellulose, Kyle Ward, JR. In Cellulose and Cellulose Derivatives, Part I; Ott, E., Spurlin, H.M., Eds.; Interscience Publishers Inc. New York, 1954; Vol. V, p 9-29

[7] Ultrafine Cellulose Fibers Produced by Asaia bogorensis, an Acetic Acid Bacterium, A. Kumagai et al. Biomacromolecules 2011, 12, 2815-2821

[8] Sensing the structural differences in cellulose from apple and bacterial cell wall materials by Raman and FT-IR spectroscopy. Szymanska-Chargot M, Cybulska J, Zdunek A. Sensors (Basel). 2011;11(6):5543-60.

[9] BRENDA, the enzyme information system in 2011. Nucleic Acids Res. 2011, 39: D670-D676, www.brenda-enzymes.org.

[10] A novel efficient $\beta$-glucanase from a paddy soil microbial metagenome with versatile activities. Biotechnol Biofuels. 2016 Feb 13;9:36

[11] Screening and characterization of a cellulase with endocellulase and exo-cellulase activity from yak rumen metagenome J. Mol. Catal. B 73, 104-110 (2011)

[12] Cloning and Characterization of an Endoglucanase Gene from Actinomyces sp. Korean Native Goat 40. Asian Australas. J. Anim. Sci. Vol. 29, No. 1: 126-133 January 2016.

[13] Use of dinitrosalicylic acid reagent for determination of reducing sugar. Analytical Chemistry. 1959;31(3):426-428.

**Claims**

1. A solid oral composition for targeted release in an intestine of a mammal comprising a core and a coating completely surrounding the core, wherein the core comprises an entrapped material to be released in the intestine, and wherein the coating or part of the coating comprises a first and a second component, wherein the first component is resistant to the environment in the stomach of a mammal and the second component enzymatically digest the first component when subjected to the more basic environment of the intestine compared to the more acidic environment of the stomach.

2. The composition of claim 1 wherein the coating is adapted to resist breakdown from the environment in a stomach of a mammal.

3. The composition of any one of the preceding claims wherein the coating prevents the release of the entrapped material in a stomach of a mammal.

4. The composition of any one of the preceding claims wherein the coating protects the entrapped material in a stomach of a mammal.

5. The composition of any one of the preceding claims wherein the coating is adapted to release the entrapped material in an intestine of a mammal.

6. The composition of any one of the preceding claims where the digesting activity of the second component is inhibited in the environment in the stomach of a mammal.

7. The composition of any one of the preceding claims wherein the entrapped material is a medicinal product, such as a protein, an enzyme, a polypeptide, an oligopeptide, a peptide, a deoxyribonucleic acid (DNA), a ribonucleic acid (RNA), a small organic molecule of less than 900 Da, or a pro-drug of any one of these materials, is a nutritional supplement, or a microbial culture, or a microbial additive, or a vaccine.

8. The composition of any one of the preceding claims wherein the first component and the second component are mixed.

9. The composition of any one of the preceding claims wherein the first component and the second component are in different layers with the first component being the outer layer and the second component the inner layer around the core.

10. The composition of any one of the preceding claims wherein the pair of the first and second component is selected from cellulose and cellulase, pectin and pectinolytic enzymes, hemicellulose and hemicellulase, lignin and lignin degrading enzymes, and fructan and fructan degrading enzymes.

11. The composition of any one of the preceding claims wherein the pair of the first and second component is selected from structurally ordered cellulose I such as bacterial cellulose or derivatives thereof and a cellulase, such as cellulase (EC 3.2.1.4) (endocellulase), cellubiase (EC 3.2.1.21) (beta-glucosidase), 1,4-beta-cellobiosidase (EC 3.2.1.91) (Exocellulase), Cellulose 1,4-beta-cellobiosidase (reducing end) (EC 3.2.1.176) (exocellulase) as well as cellulase complexes and mixtures thereof.

12. The composition of any one of the preceding claims wherein the second component is enzymatically active and digests the first component in the environment in the intestine of a mammal.

13. The composition of claim 12 wherein the second component has maximum enzymatic activity in the range pH 3-12, such as pH 3.5 to 9.5, or 3.0 to 9.0, or 4.0 to 9.0.

14. The composition of any one of the preceding claims wherein the composition is a tablet or a capsule or other type of solid oral dosage form.

15. The composition of any one of claims 1-14, wherein the entrapped material is selected from:

a) Proteins, Human growth hormone (hGH), Calcitonin, Insulin, GLP-1 analogues, GLP-1
b) Peptides, Octreotide
c) Oral vaccines, Oral cholerae vaccine, Mycoplasma hyopneumoniae oral vaccine, Live bacterial cells as attenuated vaccines, live cell culture.
d) Small organic molecules 200<MW<900 g/mol, Desmopressin, Vasopressin, Cyclo-sporine, Ranitidine, Diclofenac, Ketoprofen, Amifostine, Omeprazole, Gemcitabine, Domperidone, Paclitaxel, Cinnarizine, Donepezil, Leucovorin, Raloxifene, Indomethacin, Dextromethorphan, Nizatidine, Peptide Val-Leu-Pro-Val-pro-Arg (VLPVPR), Flurbiprofen, Mebendazole, Thymidine, Zolpidem tartarate, Loratidine, Venlafaxine, Tamsulosin, Urapidil, Prednisolone, Miconazole, Diltiazem, Ambroxol, Captopril, Acyclovir, Cimetidine, Metoprolol, Griseofulvin, Atazanavir, Ibuprofen, Azithromycin, Lercanidipine, Sulfacetamide, Azelastine, Zidovudine, Cloricromene, Oxymatrine, Acarbose, Propranolol, Alfuzosin, Stavudine, Lobenzarit, Genistein, Verapamil, Terbinafine, Lomoxicam, Clotrimazole.

16. Use of a pair of components for preparing a coating for a solid oral dosage form where the coating is degraded when subjected to a pH change from a lower to a higher pH, wherein the digestion of the first component by the second component is inhibited at the lower pH and the second component digest the first component when subjected to the higher pH.

**Patentansprüche**

1. Eine feste orale Zusammensetzung zur gezielten Freisetzung in einem Darm eines Säugertiers, umfassend einen Kern und eine den Kern vollständig umgebende Beschichtung, wobei der Kern ein eingeschlossenes Material umfasst, das in den Darm freigesetzt werden soll, und wobei die Beschichtung oder ein Teil der Beschichtung eine erste und eine zweite Komponente umfasst, wobei die erste Komponente gegenüber der Umgebung in dem Magen eines Säugertiers resistent ist und die zweite Komponente die erste Komponente enzymatisch verdaut, wenn sie der basischeren Umgebung des Darms im Vergleich zu der saureren Umgebung des Magens ausgesetzt wird.

2. Die Zusammensetzung von Anspruch 1, wobei die Beschichtung angepasst ist, um einem Zusammenbruch aus der Umgebung in einem Magen eines Säugertiers zu widerstehen.

3. Die Zusammensetzung von einem der vorhergehenden Ansprüche, wobei die Beschichtung die Freisetzung des eingeschlossenen Materials in einem Magen eines Säugertiers verhindert.

4. Die Zusammensetzung von einem der vorhergehenden Ansprüche, wobei die Beschichtung das eingeschlossene Material in einem Magen eines Säugertiers schützt.

5. Die Zusammensetzung von einem der vorhergehenden Ansprüche, wobei die Beschichtung angepasst ist, um das eingeschlossene Material in einem Darm eines Säugertiers freizusetzen.

6. Die Zusammensetzung von einem der vorhergehenden Ansprüche, wobei die Verdauungsaktivität der zweiten Komponente in der Umgebung im Magen eines Säugertiers gehemmt wird.

7. Die Zusammensetzung von einem der vorhergehenden Ansprüche, wobei das eingeschlossene Material ein medizinisches Produkt, wie ein Protein, ein Enzym, ein Polypeptid, ein Oligopeptid, ein Peptid, eine Desoxyribonukleinsäure (DNA), eine Ribonukleinsäure (RNA), ein kleines organisches Molekül von weniger als 900 Da oder ein Prodrug von einem von diesen Materialien ist, ein Nahrungsergänzungsmittel oder eine mikrobielle Kultur oder ein mikrobieller Zusatzstoff oder ein Impfstoff ist.

8. Die Zusammensetzung von einem der vorhergehenden Ansprüche, wobei die erste Komponente und die zweite Komponente gemischt sind.

**9.** Die Zusammensetzung von einem der vorhergehenden Ansprüche, wobei die erste Komponente und die zweite Komponente in verschiedenen Schichten vorliegen, wobei die erste Komponente die äußere Schicht und die zweite Komponente die innere Schicht um den Kern herum ist.

**10.** Die Zusammensetzung von einem der vorhergehenden Ansprüche, wobei das Paar der ersten und zweiten Komponente ausgewählt ist aus Cellulose und Cellulase, Pektin und pektinolytischen Enzymen, Hemicellulose und Hemicellulase, Lignin und Lignin abbauenden Enzymen und Fructan und Fructan abbauenden Enzymen.

**11.** Die Zusammensetzung von einem der vorhergehenden Ansprüche, wobei das Paar der ersten und zweiten Komponente ausgewählt ist aus strukturell geordneter Cellulose I, wie bakterielle Cellulose oder Derivate davon, und einer Cellulase, wie Cellulase (EC 3.2.1.4) (Endocellulase), Cellubiase (EC 3.2.1.21) (Beta-Glucosidase), 1,4-beta-Cellobiosidase (EC 3.2.1.91) (Exocellulase), Cellulose 1,4-beta-Cellobiosidase (reduzierendes Ende) (EC 3.2.1.176) (Exocellulase) sowie Cellulase-Komplexen und Mischungen davon.

**12.** Die Zusammensetzung von einem der vorhergehenden Ansprüche, wobei die zweite Komponente enzymatisch aktiv ist und die erste Komponente in der Umgebung im Darm eines Säugertiers verdaut.

**13.** Die Zusammensetzung von Anspruch 12, wobei die zweite Komponente eine maximale enzymatische Aktivität im Bereich von pH 3-12, wie etwa von pH 3,5 bis 9,5 oder von 3,0 bis 9,0 oder von 4,0 bis 9,0, hat.

**14.** Die Zusammensetzung von einem der vorhergehenden Ansprüche, wobei die Zusammensetzung eine Tablette oder eine Kapsel oder eine andere Art von fester oraler Arzneiform ist.

**15.** Die Zusammensetzung nach einem der Ansprüche 1 bis 14, wobei das eingeschlossene Material ausgewählt ist aus:

a) Proteinen, menschlichem Wachstumshormon (hGH), Calcitonin, Insulin, GLP-1-Analoga, GLP-1
b) Peptiden, Octreotid
c) oralen Impfstoffen, oralem Cholera-Impfstoff, oralem Mycoplasma hyopneumoniae-Impfstoff, lebenden Bakterienzellen als attenuierten Impfstoffen, lebender Zellkultur.
d) kleinen organischen Molekülen 200<MW<900 g/mol, Desmopressin, Vasopressin, Cyclosporin, Ranitidin, Diclofenac, Ketoprofen, Amifostin, Omeprazol, Gemcitabin, Domperidon, Paclitaxel, Cinnarizin, Donepezil, Leucovorin, Raloxifen, Indomethacin, Dextromethorphan, Nizatidin, Peptid Val-Leu-Pro-Val-pro-Arg (VLPVPR), Flurbiprofen, Mebendazol, Thymidin, Zolpidemtartarat, Loratidin, Venlafaxin, Tamsulosin, Urapidil, Prednisolon, Miconazol, Diltiazem, Ambroxol, Captopril, Acyclovir, Cimetidin, Metoprolol, Griseofulvin, Atazanavir, Ibuprofen, Azithromycin, Lercanidipin, Sulfacetamid, Azelastin, Zidovudin, Cloricromen, Oxymatrin, Acarbose, Propranolol, Alfuzosin, Stavudin, Lobenzarit, Genistein, Verapamil, Terbinafin, Lornoxicam, Clotrimazol.

**16.** Verwendung eines Paars von Komponenten zur Herstellung einer Beschichtung für eine feste orale Arzneiform, wo die Beschichtung abgebaut wird, wenn sie einer pH-Änderung von einem niedrigeren zu einem höheren pH ausgesetzt wird, wobei die Verdauung der ersten Komponente durch die zweite Komponente bei dem niedrigeren pH gehemmt wird und die zweite Komponente die erste Komponente verdaut, wenn sie dem höheren pH ausgesetzt wird.

## Revendications

**1.** Une composition orale solide pour une libération ciblée dans un intestin d'un mammifère comprenant un noyau et un revêtement entourant complètement le noyau, dans laquelle le noyau comprend un matériau piégé destiné à être libéré dans l'intestin, et dans laquelle le revêtement ou une partie du revêtement comprend un premier et un second composant, dans laquelle le premier composant est résistant à l'environnement dans l'estomac d'un mammifère et le second composant digère enzymatiquement le premier composant lorsqu'il est soumis à l'environnement plus basique de l'intestin par rapport à l'environnement plus acide de l'estomac.

**2.** La composition de la revendication 1, dans laquelle le revêtement est adapté pour résister à la dégradation de l'environnement dans l'estomac d'un mammifère.

**3.** La composition selon l'une quelconque des revendications précédentes, dans laquelle le revêtement empêche la libération du matériau piégé dans l'estomac d'un mammifère.

**4.** La composition de l'une quelconque des revendications précédentes, dans laquelle le revêtement protège le matériau piégé dans l'estomac d'un mammifère.

**5.** La composition de l'une quelconque des revendications précédentes, dans laquelle le revêtement est adapté pour libérer le matériau piégé dans un intestin d'un mammifère.

**6.** La composition de l'une quelconque des revendications précédentes, où l'activité de digestion du second composant est inhibée dans l'environnement dans l'estomac d'un mammifère.

**7.** La composition de l'une quelconque des revendications précédentes, dans laquelle le matériau piégé est un médicament, tel qu'une protéine, une enzyme, un polypeptide, un oligopeptide, un peptide, un acide désoxyribonucléique (ADN), un acide ribonucléique (ARN), une petite molécule organique de moins de 900 Da, ou un promédicament de l'un quelconque de ces matériaux, est un supplément nutritionnel, ou une culture microbienne, ou un additif microbien, ou un vaccin.

**8.** La composition de l'une quelconque des revendications précédentes, dans laquelle le premier composant et le second composant sont mélangés.

**9.** La composition de l'une quelconque des revendications précédentes, dans laquelle le premier composant et le second composant sont dans des couches différentes, le premier composant étant la couche extérieure et le second composant étant la couche intérieure autour du noyau.

**10.** La composition de l'une quelconque des revendications précédentes, dans laquelle la paire du premier et du second composant est choisie parmi la cellulose et la cellulase, la pectine et les enzymes pectinolytiques, l'hémicellulose et l'hémicellulase, la lignine et les enzymes dégradant la lignine, et le fructane et les enzymes dégradant le fructane.

**11.** La composition de l'une quelconque des revendications précédentes, dans laquelle la paire du premier et du second composant est choisie parmi la cellulose I ordonnée structurellement telle que la cellulose bactérienne ou des dérivés de celle-ci et une cellulase, telle que la cellulase (EC 3.2.1.4) (endocellulase), la cellubiase (EC 3.2.1.21) (bêta-glucosidase), la 1,4-bêta-cellobiosidase (EC 3.2.1.91) (exocellulase), la cellulose 1,4-bêta-cellobiosidase (extrémité réductrice) (EC 3.2.1.176) (exocellulase) ainsi que des complexes de cellulase et des mélanges de ceux-ci.

**12.** La composition de l'une quelconque des revendications précédentes, dans laquelle le second composant est actif enzymatiquement et digère le premier composant dans l'environnement dans l'intestin d'un mammifère.

**13.** La composition de la revendication 12, dans laquelle le second composant a une activité enzymatique maximale dans la plage de pH 3-12, telle qu'un pH de 3,5 à 9,5, ou de 3,0 à 9,0, ou de 4,0 à 9,0.

**14.** La composition de l'une quelconque des revendications précédentes, dans laquelle la composition est un comprimé ou une capsule ou un autre type de forme galénique orale solide.

**15.** La composition de l'une quelconque des revendications 1-14, dans laquelle le matériau piégé est choisi de :

   a) Protéines, hormone de croissance humaine (hGH), calcitonine, insuline, analogues du GLP-1, GLP-1
   b) Peptides, octréotide
   c) Vaccins oraux, vaccin oral contre le choléra, vaccin oral contre Mycoplasma hyopneumoniae, cellules bactériennes vivantes en tant que vaccins atténués, culture de cellules vivantes.
   d) Petites molécules organiques 200<MW<900 g/mol, Desmopressine, Vasopressine, Cyclosporine, Ranitidine, Diclofénac, Kétoprofène, Amifostine, Oméprazole, Gemcitabine, Dompéridone, Paclitaxel, Cinnarizine, Donépézil, Leucovorine, Raloxifène, Indométhacine, Dextrométhorphane, Nizatidine, Peptide Val-Leu-Pro-Val-pro-Arg (VLPVPR), Flurbiprofène, Mébendazole, Thymidine, Zolpidem tartrate, Loratidine, Venlafaxine, Tamsulosine, Urapidil, Prednisolone, Miconazole, Diltiazem, Ambroxol, Captopril, Acyclovir, Cimétidine, Métoprolol, Griséofulvine, Atazanavir, Ibuprofène, Azithromycine, Lercanidipine, Sulfacétamide, Azélastine, Zidovudine, Cloricromène, Oxymatrine, Acarbose, Propranolol, Alfuzosine, Stavudine, Lobenzarit, Genistein, Vérapamil, Terbinafine, Lornoxicam, Clotrimazole.

**16.** Utilisation d'une paire de composants pour préparer un revêtement pour une forme galénique orale solide, où le revêtement est dégradé lorsqu'il est soumis à un changement de pH d'un pH inférieur à un pH supérieur, dans laquelle la digestion du premier composant par le second composant est inhibée au pH inférieur et le second composant digère le premier composant lorsqu'il est soumis au pH supérieur.

## Figure 1

**Small intestine**

Esophagus | Stomach | Duodenum | Jejunum | Ileum | Colon

Transit time: Seconds

pH 6-7

Digesting enzymes

Transit time: minutes to 5.5 hours

pH ≤ 6.0 at meal
pH ≤ 3.5 45 min after meal
pH ≤ 1.5 120 min after meal

Gastric fluid:
Pepsin and other digesting enzymes

Transit time: 2.5 – 11 hours

pH 5 – 7

## Figure 2

Produce cellulase X (CX)

Purify CX

Produce bacterial cellulose (BC) membrane

Purify wet BC membrane

Infuse CX into the BC membrane

Dry membrane yielding sheets of BC with infused CX

Embed tablet in sheets of BC with infused CX

Key Process Parameters:        Ratio CX(act. (U))/BC (g)    Specific weight (g/cm²) of dry membrane    Seal tightness

Figure 3

Figure 4

Figure 5

Stomach transit time 127 min.    Small intestine transit time 162 min.

Time (min)

Figure 6

Stomach transit
time 60 min

Small intestine transit time 275 min

Time (min)

Figure 7

Figure 8

Stomach transit time 337 min

Small intestine transit time 162 min

Time (min)

Figure 9

Stomach transit
time 5 min

Time (min)

Small intestine transit time 669 min

Figure 10

Stomach transit time 337 min  Small intestine transit time 669 min

Time (min)

Figure 11

60 min post meal
ingression

Small intestine transit time 275 min

Time (min)

## Figure 12

Long stomach transit time 337 min    Short small intestine transit time 162 min

Time (min)

## Figure 13

Long stomach transit time 337 min    Short small intestine transit time 162 min

Time (min)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016199303 A1
- WO 2017168426 A1
- WO 2008150426 A1

**Non-patent literature cited in the description**

- EVALUATION OF AN ENZYME-CONTAINING CAPSULAR SHAPED PULSATI LE DRUG DELIVERY SYSTEM. **KROEGEL I et al.** PHARMACEUTICAL RESEARCH. SPRINGER NEW YORK LLC, 01 January 1999, vol. 16, 1424-1429
- **DRESSMAN JB ; BERARDI RR ; DERMENT-ZOGLOU LC ; RUSSELL TL ; JARVENPAA KM.** Upper Gastrointestinal (GI) pH in Young, Healthy Men and Women. *Pharm Res,* 1990, vol. 7, 756-761
- **CLARYSSE S ; TACK J ; LAMMERT F ; DUCHATEAU G ; REPPAS C ; AUGUSTIJNS P.** Postprandial Evolution in Composition and Characteristics of Human Duodenal Fluids in Different Nutritional States. *J. Pharm Sci,* 2009, vol. 98, 1177-1192
- **ZARATE N ; MOHAMMED SD ; O'SHAUGHNESSY E ; NEWELL M ; YAZAKI E ; WILLIAMS NS ; LUNNISS PJ ; SEMLER JR ; SCOTT SM.** Accurate localization of a fall in pH within the ileocecal region: Validation using a dual-scintigraphic technique. *Am J Physiol - Gastroint and Liver Physiol,* 2010, vol. 299 (6), G1276-G1286
- **WORSØE et al.** Gastric transit and small intestinal transit time and motility assessed by a magnet tracking system. *BMC Gastroenterology,* 2011, vol. 11, 145

- **I. SAROSIEK et al.** Assessment of regional gut transit times in healthy controls and patients with gastroparesis using wireless motility technology. *Aliment Pharmacol Ther,* 15 January 2010, vol. 31 (2), 313-322
- Occurrence of cellulose. **KYLE WARD, JR.** Cellulose and Cellulose Derivatives. Interscience Publishers Inc, 1954, vol. V, 9-29
- **A. KUMAGAI et al.** Ultrafine Cellulose Fibers Produced by Asaia bogorensis, an Acetic Acid Bacterium. *Biomacromolecules,* 2011, vol. 12, 2815-2821
- **BRENDA.** the enzyme information system in 2011. *Nucleic Acids Res.,* 2011, vol. 39, D670-D676, www.brenda-enzymes.org
- A novel efficient β-glucanase from a paddy soil microbial metagenome with versatile activities. *Biotechnol Biofuels,* 13 February 2016, vol. 9, 36
- Screening and characterization of a cellulase with endocellulase and exo-cellulase activity from yak rumen metagenome. *J. Mol. Catal. B,* 2011, vol. 73, 104-110
- Cloning and Characterization of an Endoglucanase Gene from Actinomyces sp. Korean Native Goat 40. Asian Australas. *J. Anim. Sci.,* January 2016, vol. 29 (1), 126-133
- Use of dinitrosalicylic acid reagent for determination of reducing sugar. *Analytical Chemistry,* 1959, vol. 31 (3), 426-428